# EUROPEAN PATENT APPLICATION

(11) **EP 2 006 290 A1**
(43) Date of publication of application: **24.12.2008**
(21) Application number: 07739783.4
(22) Date of filing: 27.03.2007
(51) Int. Cl.: C07D 477/00, C07D 487/04

(54) **IMPROVED PROCESS FOR PRODUCING CARBAPENEM COMPOUND**

(30) Priority: 28.03.2006 JP 2006089254
(71) Applicant: Kaneka Corporation, Kita-ku Osaka-shi, Osaka 530-8288 (JP)
(72) Inventor: NISHINO, Keita, Takasago-shi, Hyogo 6768688 (JP); KOGA, Teruyoshi, Takasago-shi, Hyogo 6768688 (JP)
(74) Representative: Hart-Davis, Jason
(86) International application number: PCT/JP2007/056345
(87) International publication number: WO 2007/111328

(57) **Abstract**

The present invention has its object to provide an easy process for producing (4R,5S,6S)-3-[[(3S,5S)-5-(dimethylaminocarbonyl)-3-pyrrolid inyl]thio]-6-[(1R)-1-hydroxyethyl]-4-methyl-7-oxo-1-azabicy clo[3.2.0]hept-2-ene-2-carboxylic acid, excellent in antimicrobial activity. The present invention relates to a process for continuously producing (4R,5S,6S)-3-[[(3S,5S)-5-(dimethylaminocarbonyl)-3-pyrrolid inyl]thio]-6-[(1R)-1-hydroxyethyl]-4-methyl-7-oxo-1-azabicy clo[3.2.0]hept-2-ene-2-carboxylic acid without isolating/purifying the reaction intermediate.

## Description

### TECHNICAL FIELD

The present invention relates to an industrially advantageous process for producing a carbapenem compound (4).

The carbapenem compound (4) is represented by the general formula (4).

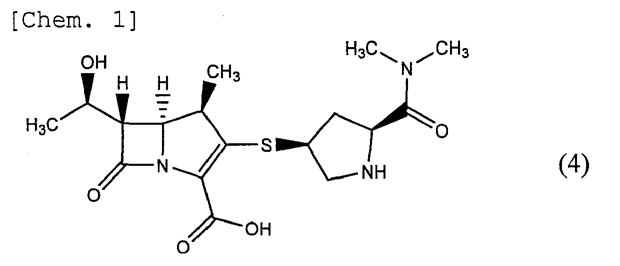

### BACKGROUND ART

Known in the art as processes for producing the compound (4) are the process which comprises reacting a compound (1) represented by the general formula (1):

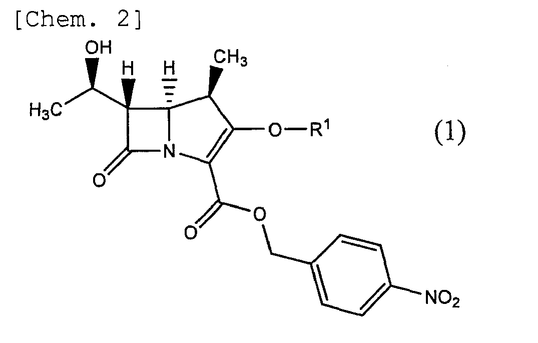

with a compound (2) represented by the general formula (2):

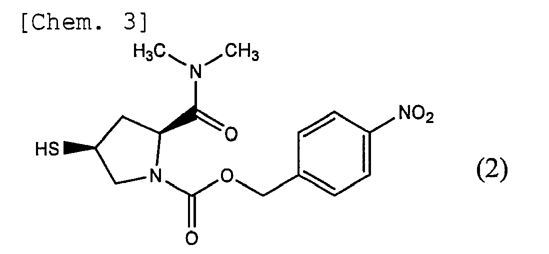

in the presence of a base to synthesize a compound (3) represented by the general formula (3):

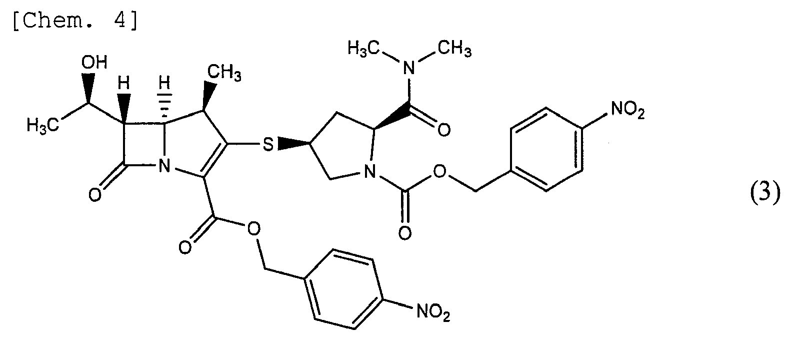

and, after such after-treatments as extraction, washing and concentration, isolating the compound (3) by chromatography, followed by deprotection (Patent Document 1) and the process which comprises reacting the compound (1) with the compound (2) in the presence of a base to synthesize the compound (3) and, after such after-treatments as extraction, washing and concentration, isolating the compound (3) by crystallization and filtration, followed by deprotection (Patent Document 2).
Patent Document 1: Japanese Kokai Publication S60-104088
Patent Document 2: WO2005/118586

### SUMMARY OF THE INVENTION

To synthesize the compound (4) from the compound (3), the deprotection is generally carried out by hydrogenation using such a heterogeneous catalyst as palladium-on-carbon (hereinafter sometimes referred to also as "Pd/C"). Generally, such a noble metal catalyst as Pd/C is susceptible to catalyst poisoning by a thiol (SH) group-containing compound, so that problems readily arise, for example marked reductions in catalytic activity.

When the compound (3) is synthesized by the reaction between the compound (1) and the compound (2), the compound (2), which is a thiol compound, often remains in the reaction mixture. In such a case, for subjecting the compound (3) to hydrogenation reaction, it is necessary to separate the compound (3) from the compound (2), and isolation of the compound (3) has been considered to be an effective means. Therefore, according to the above-cited Patent Document 1 and Patent Document 2, the synthetic intermediate compound (3) is isolated.

In the above-cited Patent Document 1, the compound (3) is isolated by column chromatography. However, it is difficult to utilize column chromatography in commercial-scale production. On the other hand, the above-cited Patent Document 2 describes crystallization as the method of isolating the compound (3). However, the crystallization step requires a long period of time, namely 72 hours or longer, so that the method is unsatisfactory from the viewpoint of productivity in commercial-scale production.

The task to be accomplished by the present invention is to find out a process for producing the compound (4), which is high in productivity and can be carried out on a commercial scale.

As a result of intensive investigations made by the present inventors, it was found that when the compound (3) is subjected, in the form of a solution in an organic solvent, without isolation thereof, to hydrogenation reaction, the compound (4) can be obtained in high yields and as a product of high quality. Such finding has led to completion of the present invention.

That is, the present invention relates to a process for producing a carbapenem compound represented by the above formula (4), the process comprising:
the step (A) of reacting a compound represented by the above formula (1) with a compound represented by the above formula (2) in an organic solvent in the presence of a base to obtain a compound represented by the above formula (3); and
the step (B) of deprotecting the compound represented by the above formula (3) to obtain the compound represented by the above formula (4),
wherein the steps (A) and (B) are carried out without isolating the compound represented by the above formula (3).

### DETAILED DESCRIPTION OF THE INVENTION

In the following, the invention is described in detail.

The term "lower" as used herein means that the number of carbon atoms is 1 to 7, preferably 1 to 4. The term "solution of the compound (3) in an organic solvent" as used herein means a liquid containing the compound (3) and an organic solvent as the components thereof and the liquid includes not only a homogeneous solution but also a slurry or suspension.

First, the step (A) is described.

The step (A) is a step of reacting a compound represented by the general formula (1):

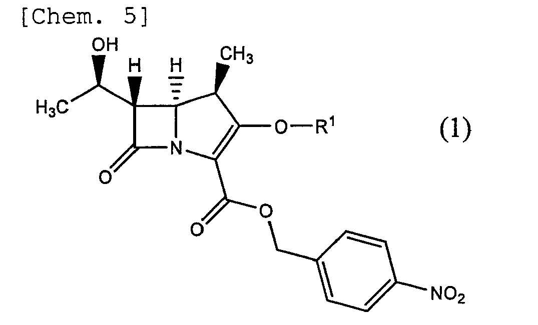

(wherein R¹ represents an acyl group)

with a compound represented by the general formula (2):

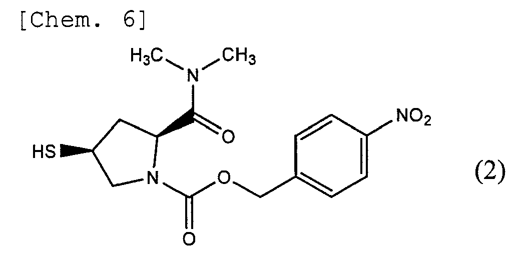

in an organic solvent in the presence of a base to obtain a compound represented by the general formula (3).

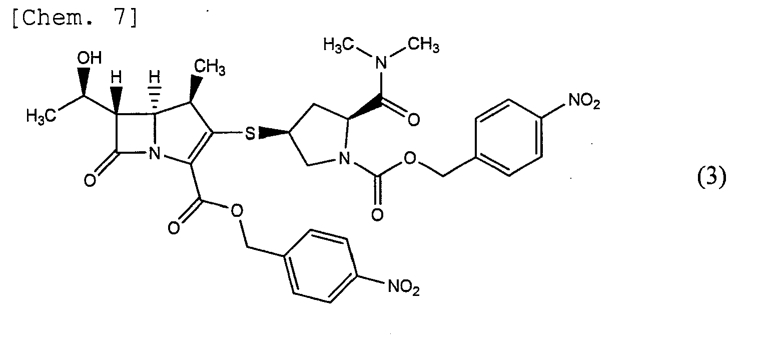

In the compound (1) to be used in this step, R¹ is an acyl group. The term "acyl group" as used herein means a group resulting from removal of one or more hydroxyl groups from an oxo acid. Therefore, the "acyl group" includes, within the meaning thereof, not only carboxylic acid-derived acyl (RCO-) groups in a narrow sense of the term but also all groups derived from carbonic acids, sulfonic acids, phosphoric acids and carbamic acids, or derivatives thereof, by removal of one or more hydroxyl groups therefrom.

As the acyl group R¹, there may be mentioned such acyl groups derived from carboxylic acids, carbonic acids, sulfonic acids, phosphoric acids and carbamic acids as aliphatic group-substituted acyl groups, alicyclic group-substituted acyl groups, aromatic group-substituted acyl groups, heterocyclic group-substituted acyl groups and acyl groups having an aromatic or heterocyclic group-substituted aliphatic group.

Specific examples of such acyl groups are given below. As the aliphatic or alicyclic group-substituted acyl groups, there may be mentioned, for example, alkanoyl groups such as formyl, acetyl, propionyl, butyryl, isobutyryl, valeryl, isovaleryl, pivaloyl and hexanoyl; alkylsulfonyl groups such as mesyl, ethylsulfonyl, propylsulfonyl, isopropylsulfonyl, butylsulfonyl, isobutylsulfonyl, pentylsulfonyl and hexylsulfonyl; N-alkylcarbamoyl groups such as methylcarbamoyl and ethylcarbamoyl; alkoxycarbonyl groups such as methoxycarbonyl, ethoxycarbonyl, propoxycarbonyl, butoxycarbonyl and tert-butoxycarbonyl; alkenyloxycarbonyl groups such as vinyloxycarbonyl and allyloxycarbonyl; alkenoyl groups such as acryloyl, methacryloyl and crotonoyl; cycloalkanecarbonyl groups such as cyclopropanecarbonyl, cyclopentanecarbonyl and cyclohexanecarbonyl; dialkoxyphosphoryl groups such as a diethoxyphosphoryl group; and so forth.

As the aromatic group-substituted acyl groups, there may be mentioned, for example, aroyl groups such as benzoyl, toluoyl and xyloyl; N-arylcarbamoyl groups such as N-phenylcarbamoyl, N-tolylcarbamoyl and N-naphthylcarbamoyl; arenesulfonyl groups such as benzenesulfonyl and tosyl; diaryloxyphosphoryl groups such as a diphenyloxyphosphoryl group; and so forth.

As the heterocyclic group-substituted acyl groups, there may be mentioned, for example, heterocyclic group-substituted carbonyl groups such as furoyl, thenoyl, nicotinoyl, isonicotinoyl, thiazolylcarbonyl, thiadiazolylcarbonyl and tetrazolylcarbonyl, and so forth.

As the acyl groups having an aromatic group-substituted aliphatic group, there may be mentioned aralkanoyl groups such as phenylacetyl, phenylpropionyl and phenylhexanoyl; aralkoxycarbonyl groups such as benzyloxycarbonyl and phenethyloxycarbonyl; aryloxyalkanoyl groups such as phenoxyacetyl and phenoxypropionyl; and so forth.

As the acyl groups having a heterocyclic group-substituted aliphatic group, there may be mentioned heterocyclic group-substituted alkanoyl groups such as thienylacetyl, imidazolylacetyl, furylacetyl, tetrazolylacetyl, thiazolylacetyl, thiadiazolylacetyl, thienylpropionyl and thiadiazolylpropionyl, and so forth.

These acyl groups may further be substituted by one or more appropriate substituents selected from among lower alkyl groups such as methyl, ethyl, propyl, isopropyl, butyl, pentyl and hexyl; halogens such as chlorine, bromine, iodine and fluorine; lower alkoxy groups such as methoxy, ethoxy, propoxy, isopropoxy, butoxy, pentyloxy and hexyloxy; lower alkylthio groups such as methylthio, ethylthio, propylthio, isopropylthio, butylthio, pentylthio and hexylthio; a nitro group and so forth. As preferred acyl groups having such a substituent, there may be mentioned mono(or di or tri)haloalkanoyl groups such as chloroacetyl, bromoacetyl, dichloroacetyl and trifluoroacetyl; mono(or di or tri)haloalkoxycarbonyl groups such as chloromethoxycarbonyl, dichloromethoxycarbonyl and 2,2,2-trichloroethoxycarbonyl; nitro-, halo- or lower alkoxyaralkoxycarbonyl groups such as nitrobenzyloxycarboyl, chlorobenzyloxycarbonyl and methoxybenzyloxycarbonyl; mono (or di or tri) haloalkylsulfonyl groups such as fluoromethylsulfonyl, difluoromethylsulfonyl, trifluoromethylsulfonyl and trichloromethylsulfonyl; and so forth.

Among the acyl groups mentioned above, diaryloxyphosphoryl groups or dialkoxyphosphoryl groups are preferred and, in particular, the diphenyloxyphosphoryl group is preferred.

Generally, the reaction between the compound (2), which is a thiol compound, and the compound (1) can be carried out in the presence of an organic base or inorganic base.

As the organic base or inorganic base to be used, there may be mentioned, for example, alkali metals such as lithium, sodium and potassium; alkaline earth metals such as calcium; alkali metal hydrides such as sodium hydride; alkaline earth metal hydrides such as calcium hydride; alkali metal hydroxides such as sodium hydroxide and potassium hydroxide; alkali metal carbonates such as sodium carbonate and potassium carbonate; alkali metal hydrogencarbonates such as sodium hydrogencarbonate and potassium hydrogencarbonate; alkali metal alkoxides such as sodium methoxide, sodium ethoxide and potassium tert-butoxide; alkali metal alkanoates such as sodium acetate; alkaline earth metal carbonates such as magnesium carbonate and calcium carbonate; di- or trialkylamines such as trimethylamine, triethylamine, N,N-diisopropylethylamine and N,N-diisopropylamine; pyridine compounds such as pyridine, picoline, lutidine, collidine, and N,N-dialkylaminopyridines like N,N-dimethylaminopyridine; quinoline; N-alkylmorpholines such as N-methylmorpholine; N,N-dialkylbenzylamines such as N,N-dimethylbenzylamine; 1,1,3,3-tetramethylguanidine; 1,8-diazabicyclo[5.4.0]undec-7-ene (DBU); 1,5-diazabicyclo[4.3.0]non-5-ene (DBN); 1,4-diazabicyclo[2.2.2]octane (DABCO) and so forth.

Among the bases mentioned above, organic bases are preferred, and amines are more preferred. Preferred as the amines are trialkylamines and, in particular, triethylamine and N,N-diisopropylethylamine are preferred.

The amount of the base to be used is not critical but, generally, the base can be used in an amount of 1.0 to 5.0 moles, preferably 1.0 to 4.0 moles, per mole of the compound of formula (1).

Generally, the compound (2) can be used in an amount of 0. 9 to 3. 0 moles, preferably 0. 95 to 2. 0 moles, more preferably 0.99 to 1.5 moles, per mole of the compound of formula (1).

The reaction temperature is not particularly restricted but the lower limit value is preferably not lower than -40°C, more preferably not lower than -30°C. The upper limit is preferably not higher than 30°C, more preferably not higher than 20°C.

The reaction time is not particularly restricted but generally is 5 minutes to 30 hours, preferably 10 minutes to 20 hours.

The reaction pressure is not particularly restricted, either. Generally, the reaction can be carried out at 1 atm (1.013 × 10⁵ Pa).

Further, the above reaction can also be carried out using an inert organic solvent, for example, a solvent properly selected from among alcohols such as methanol, ethanol, n-propanol, isopropanol, n-butanol, 2-butanol and n-pentanol; ethers such as tetrahydrofuran, diethyl ether and dioxane; esters such as methyl acetate, ethyl acetate, n-propyl acetate, isopropyl acetate, n-butyl acetate, sec-butyl acetate, isobutyl acetate and tert-butyl acetate; halogenated hydrocarbons such as dichloromethane, chloroform and carbon tetrachloride; aromatic hydrocarbons such as toluene; ketones such as acetone, 2-butanone, 3-methyl-2-butanone, 2-pentanone, 4-methyl-2-pentanone and 2-hexanone; amides such as N,N-dimethylformamide, N,N-dimethylacetamide, N-methylpyrrolidone and N-ethylpyrrolidone; and nitriles such as acetonitrile, or a mixed solvent of these.

Among the solvents mentioned above, tetrahydrofuran, ethyl acetate, dichloromethane, acetone, 2-butanone, 4-methyl-2-pentanone, N,N-dimethylformamide, N,N-dimethylacetamide, N-methylpyrrolidone, N-ethylpyrrolidone, acetonitrile, or a mixed solvent thereof can be suitably used; a nitrile and/or an amide is more suitable. Specifically, there may be mentioned acetonitrile, N,N-dimethylformamide, N,N-dimethylacetamide, N-methylpyrrolidone and N-ethylpyrrolidone, or a mixed solvent of these. Among them, acetonitrile, N,N-dimethylformamide, N,N-dimethylacetamide and N-methylpyrrolidone are particularly preferred.

The amount of the solvent to be used in the above reaction is not particularly restricted but, generally, the weight proportion of the solvent is 1 to 30 times, preferably 2 to 20 times, the weight of the compound of formula (1).

The above reaction gives a solution of the compound (3) in the organic solvent.

If necessary, the organic solvent solution of the compound (3) obtained in the step (A) can be subjected to the step of washing with water in the presence of an organic solvent capable of separating from water to form two phases.

When the organic solvent solution of the compound (3) obtained in the step (A) can separate from water to form two phases, the washing step can be carried out without newly adding an organic solvent; considering the efficiency of washing, however, the same or a different organic solvent may be added. It is of course possible to add a new organic solvent if the organic solvent solution of the compound (3) obtained in the step (A) cannot separate from water. The organic solvent to be added can be properly selected depending on the solvent species forming the organic solvent solution of the compound (3), the solubility of the compound (3) and other factors; as such, there may be mentioned, for example, alcohols such as n-butanol, n-pentanol and 2-butanol; ethers such as tetrahydrofuran, diethyl ether and dioxane; esters such as methyl acetate, ethyl acetate, n-propyl acetate, isopropyl acetate, n-butyl acetate, sec-butyl acetate, isobutyl acetate and tert-butyl acetate; halogenated hydrocarbons such as dichloromethane, chloroform and carbon tetrachloride; aromatic hydrocarbons such as toluene; ketones such as 2-butanone, 3-methyl-2-butanone, 2-pentanone, 4-methyl-2-pentanone and 2-hexanone; and so forth. Among the solvents mentioned above, n-butanol, tetrahydrofuran, ethyl acetate, isopropyl acetate, dichloromethane, 2-butanone, 4-methyl-2-pentanone, or a mixed solvent of these can suitably be used. More preferred are ethyl acetate, dichloromethane and 2-butanone.

The amount of the organic solvent to be used for the above washing is not particularly restricted provided that separation into an organic solvent phase and an aqueous phase can be effected; generally, however, the weight proportion thereof is 1 to 30 times, preferably 2 to 20 times, the weight of the compound of formula (1).

The water to be used for the above washing may contain an acid, base or salt, for instance. The amount of water to be used is not particularly restricted provided that separation into an aqueous phase and an organic solvent phase can be effected; generally, however, the weight proportion thereof is 1 to 20 times, preferably 2 to 10 times, the weight of the compound of formula (1). The frequency of washing with water is not particularly restricted but the washing may be repeated a plurality of times.

The above-mentioned washing can be done within a temperature range from the temperature at which the aqueous phase is not frozen to the boiling point of the organic solvent and, generally, can be carried out at -15 to 30°C, preferably -10 to 25°C.

Prior to the submission thereof to the next step (B), the organic solvent solution of the compound (3) may be subjected to the above-mentioned washing step or drying, concentration, adsorption treatment (e.g. treatment with activated carbon) and/or filtration, among others, if necessary.

In the case of commercial-scale practice, it is preferred, from the productivity viewpoint, that the organic solvent solution of the compound (3) as obtained in the step (A) be subjected to the above-mentioned washing step and then, as such, subjected to the next step, without carrying out any other after-treatment.

The step (B) is a step of deprotecting the compound represented by the formula (3) to give a carbapenem compound.

The carbapenem compound is represented by the general formula (4).

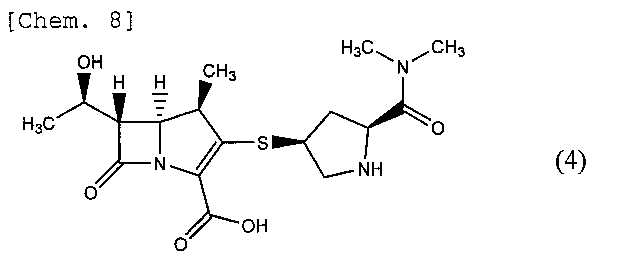

According to the present invention, the steps (A) and (B) are carried out without isolating the compound (3) and, therefore, in the step (B), the organic solvent solution of the compound (3) as obtained in the step (A) may be used as such or the water-containing organic solvent solution of the compound (3) as coming from the above-mentioned washing step may be used or an organic solvent solution of the compound (3) as resulting from solvent substitution following concentration or the like may be used. The organic solvent to be used is not particularly restricted but includes the same ones as those to be used in the step (A). Specifically, the solvent may be properly selected from among alcohols such as methanol, ethanol, n-propanol, isopropanol, n-butanol, 2-butanol and n-pentanol; ethers such as tetrahydrofuran, diethyl ether and dioxane; esters such as methyl acetate, ethyl acetate, n-propyl acetate, isopropyl acetate, n-butyl acetate, sec-butyl acetate, isobutyl acetate and tert-butyl acetate; halogenated hydrocarbons such as dichloromethane, chloroform and carbon tetrachloride; aromatic hydrocarbons such as toluene; ketones such as acetone, 2-butanone, 3-methyl-2-butanone, 2-pentanone, 4-methyl-2-pentanone and 2-hexanone; amides such as N,N-dimethylformamide, N,N-dimethylacetamide, N-methylpyrrolidone and N-ethylpyrrolidone; nitriles such as acetonitrile; and a mixed solvent of these. An organic solvent and/or water may newly be added. It goes without saying that the organic solvent solution may be a water-containing solution or a water-saturated solution or may be a two-phase system consisting of water and the organic solvent solution as separated from each other.

The organic solvent solution to be used in the step (B) is preferably a solution containing an alcohol having 3 to 8 carbon atoms and particularly preferably a solution containing n-butanol. More specifically, a mixed solvent of n-butanol and ethyl acetate may be mentioned.

The concentration of the compound (3) in the organic solvent solution to be used in the step (B) is not particularly restricted but, generally, it is not lower than 1% by weight, preferably not lower than 3% by weight, more preferably 5 to 50% by weight. On that occasion, even when the compound (3) precipitates out as a solid to form a slurry or precipitates out as an oil to form a suspension, the slurry or suspension can be used as such in the step (B), without isolation.

Since the steps (A) and (B) are conducted without isolation of the compound (3), the steps (A) and (B) can be carried out in one and the same reaction vessel. Alternatively, the deprotection reaction may be carried out in a reaction vessel different from the one used in the step (A).

The deprotection of the carboxyl group-protecting group (p-nitrobenzyl group) and the amino group-protecting group (p-nitrobenzyloxycarbonyl group) in the compound (3) can be carried out by hydrogenation or treatment with zinc in a buffer solution, for instance. Hydrogenation is particularly preferred since it can be readily carried out on a commercial scale.

In the following, the method of deprotection by hydrogenation is described in detail.

The hydrogenation-based deprotection reaction is carried out in a mixed solvent of water and such an organic solvent as mentioned above.

The amount of the organic solvent to be used in the deprotection reaction is not particularly restricted but, generally, the weight proportion thereof is 1 to 30 times, preferably 2 to 20 times, the weight of the compound of formula (3). As the organic solvent to be used in the deprotection reaction, there may be mentioned the same solvents as the inert organic solvents mentioned above referring to the step (A). It is preferred that the organic solvent contain 10 to 90% by weight, preferably 20 to 80% by weight, of an alcohol having 3 to 8 carbon atoms.

The amount of water to be used is not particularly restricted but, generally, the weight proportion thereof is 5 to 100 times, preferably 10 to 50 times, the weight of the compound of formula (3). The water to be used may contain an acid, base or salt, for instance.

Usable as the hydrogen source for hydrogenation are formic acid or a salt thereof, and hydrogen gas. From the economical viewpoint, the use of hydrogen gas is preferred. When hydrogen gas is used, it is generally preferred that the hydrogenation be carried out within a hydrogen pressure range of from atmospheric pressure (1.013 × 10⁵ Pa) to 0.5 MPa.

As for the hydrogenation catalyst, catalysts containing platinum, palladium or the like can be used; however, palladium-containing catalysts are preferred and, in particular, palladium catalysts adsorbed on active carbon are preferred.

The deprotection reaction can be properly carried out at a temperature of 0 to 50°C, preferably 15 to 40°C, for 1 minute to 5 hours; the deprotection can be suitably carried out by such treatment.

When the pH of the aqueous phase is higher than 7 or lower than 4 in the hydrogenation mentioned above, the formation of degradation products from the compound (4) tends to be promoted; therefore, the reaction is preferably carried out while adjusting the pH of the aqueous phase to 4 to 7. Such buffer solutions having a pH of 4 to 7 as acetate buffer solutions, morpholinopropanesulfonic acid-sodium hydroxide buffer solutions or phosphate buffer solutions may be used or the pH of the aqueous phase may be adjusted to 4 to 7 by addition of an acid or base thereto.

The compound (4) obtained by carrying out the step (A) and the step (B) without isolating the compound (3), as mentioned above, can be isolated and purified by subjecting the same to such ordinary after-treatments as filtration, phase separation, pH adjustment, extraction, washing, adsorption treatment (e.g. active carbon treatment), concentration, column chromatography, crystallization, and recrystallization.

By carrying out a phase separation procedure as an after-treatment, fat-soluble impurities formed in the deprotection reaction can be removed from the aqueous phase containing the compound (4). The phase separation procedure is preferably carried out after removal of the hydrogenation catalyst by a filtration procedure following completion of the deprotection reaction. When two phases, namely the aqueous phase containing the compound (4) and the organic phase are formed in the phase separation procedure, it is not necessary to add a new portion of an organic solvent; considering the washing efficiency, however, the same or a different organic solvent may be added. It goes without saying that when the mixture does not separate into the aqueous phase and organic phase, an organic solvent should be newly added. As the organic solvent to be added, there may be mentioned, for example, alcohols such as n-butanol, n-pentanol and 2-butanol; ethers such as tetrahydrofuran, diethyl ether and dioxane; esters such as methyl acetate, ethyl acetate, n-propyl acetate, isopropyl acetate, n-butyl acetate, sec-butyl acetate, isobutyl acetate and tert-butyl acetate; halogenated hydrocarbons such as dichloromethane, chloroform and carbon tetrachloride; aromatic hydrocarbons such as toluene; and ketones such as 2-butanone, 3-methyl-2-butanone, 2-pentanone, 4-methyl-2-pentanone and 2-hexanone. Among the solvents mentioned above, alcohols, esters, halogenated hydrocarbons, ketones, and mixed solvents of these can suitably be used. Preferred are alcohols, esters and halogenated hydrocarbons; more preferred are alcohols. Preferred as the alcohols are alcohols containing 3 to 8 carbon atoms, for example, n-butanol, n-pentanol and 2-butanol.

By carrying out a concentration procedure as an after-treatment, it becomes possible to remove the organic solvent from the aqueous phase containing the compound (4) and, at the same time, increase the concentration of the compound (4) through removal of water to a reasonable extent. To increase the concentration of the compound (4) is an effective after-treatment method since it leads to an improved recovery rate of crystals in the crystallization procedure which is described later herein. Since, however, the compound (4) is not very stable in aqueous solutions, the yield thereof will be lower if a high temperature and a prolonged period of time are required in the concentration procedure. Therefore, the concentration is preferably carried out using a thin-film evaporator, which can reduce the thermal history, or a reverse osmosis membrane concentrator, which does not require heating and, from the viewpoint of organic solvent removal, the concentration is more preferably carried out using a thin-film evaporator.

The aqueous solution of the compound (4) can be decolorized by carrying out an active carbon treatment as an after-treatment. The decolorization by active carbon treatment reduces the coloration of the crystals obtained by the crystallization procedure described later herein. When the above-mentioned concentration procedure is carried out to remove the organic solvent and then the active carbon treatment is carried out, the effect of decolorizing the aqueous solution is enhanced, so that the active carbon treatment is preferably carried out following the concentration procedure.

By carrying out, as an after-treatment, purification by column chromatography using a hydrophobic resin, it is possible to remove salts and highly polar impurities. In cases where salt-containing water, such as a buffer solution, is used and/or highly polar impurities are formed in an increased amount in the deprotection step (B), for instance, such salts and/or highly polar impurities may get mixed in the crystals obtained by the crystallization procedure described later herein. In such a case, the salts and highly polar impurities can be removed by hydrophobic resin column chromatography and the contamination of the crystals by them can be reduced thereby. A concentration procedure may be carried out to increase the concentration of the compound (4) after carrying out the hydrophobic resin column chromatography.

Next, the method of crystallization of the above-mentioned compound (4) is described.

By subjecting the aqueous solution of the compound (4) as obtained in the step (B) further to a crystallization step, it is possible to obtain trihydrate crystals of (4R,5S,6S)-3-[[(3S,5S)-5-(dimethylaminocarbonyl)-3-pyrrolid inyl]thio]-6-[(1R)-1-hydroxyethyl]-4-methyl-7-oxo-1-azabicy clo[3.2.0]hept-2-ene-2-carboxylic acid represented by the general formula (5).

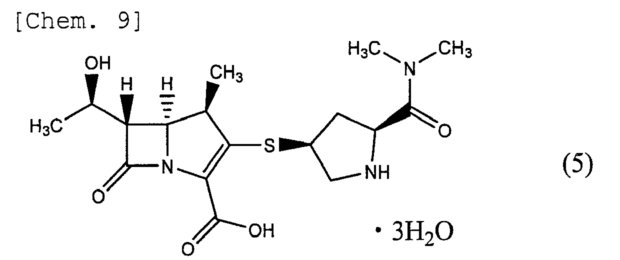

In the following, the crystallization step is described. The aqueous solution to be used is generally the aqueous solution containing the compound (4) as obtained in the step (B). It may be the one obtained after one or more of the above-mentioned after-treatments, if necessary. As for the concentration, the aqueous solution preferably contains 1 to 10% by weight, more preferably 2 to 6% by weight, of the compound (4).

As for the method of crystallization, those concentration crystallization, cooling crystallization, and poor solvent admixing crystallization methods which are commonly carried out, can be carried out either singly or in combination.

Preferred is the crystallization method comprising admixing a poor solvent to the compound represented by the general formula (5) therewith; in the following, the poor solvent admixing crystallization method is described.

The poor solvent admixing method is generally carried out by adding a poor solvent to the aqueous solution, although the aqueous solution may be added to a poor solvent.

As the above poor solvent, there may be mentioned alcohols, ketones, ethers and nitriles and, more specifically, there may be mentioned ethanol, isopropyl alcohol, acetone, tetrahydrofuran, dioxane and acetonitrile, and others. Preferred is acetone. The amount of the poor solvent to be used is preferably 0.5 to 10 volumes, particularly preferably 1 to 5 volumes, per volume of the aqueous solution. The crystallization temperature is not particularly restricted but preferably is -20 to 20°C, particularly preferably -10 to 10°C. When a poor solvent is added to the aqueous solution for crystallization, however, it is necessary for the temperature to be not lower than the temperature at which the aqueous solution is frozen.

In the crystallization step, seed crystals may be added, if necessary.

The process of the invention can be carried out continuously, without isolating/purifying the compound (3), which is the intermediate product in the reaction process mentioned above. Therefore, the invention is especially advantageous in the mass production of the compound (4) on a commercial scale.

Further, the invention is also advantageous in that the reaction steps mentioned above can be carried out in one and the same reaction vessel (namely in the one-pot manner).

### BEST MODE FOR CARRYING OUT THE INVENTION

The following examples and reference examples further illustrate the present invention. These are, however, by no means limitative of the scope of the invention.

In the following examples and reference examples, the following HPLC analysis conditions 1 were used in analyzing the compound (3), and the following HPLC analysis conditions 2 were used in analyzing the compound (4).

[HPLC analysis conditions 1]
Apparatus: Shimadzu Corporation LC-10A series
Column: GL Sciences Inc. ODS column Inertsil ODS-2 (4.6 mm x 150 mm)
Eluent: Acetonitrile/water = 45/55 (v/v)
Flow rate: 1.0 ml/min
Detection: 267 nm (UV detector)
Temperature: 25°C

[HPLC analysis conditions 2]
Apparatus: Shimadzu Corporation LC-10A series
Column: YMC Co., Ltd. ODS column YMC-Pack ODS-A A-303 (4.6 mm x 250 mm)
Eluent: Acetonitrile/phosphate buffer (pH 5.0) = 7/100 (v/v) Flow rate: 1.0 ml/min
Detection: 220 nm (UV detector)
Temperature: 40°C

### (Example 1)

### Production of p-nitrobenzyl (4R,5S,6S)-3-[[(3S,5S)-1-(p-nitrobenzyloxycarbonyl)-5-(dime thylaminocarbonyl)-3-pyrrolidinyl]thio]-6-[(1R)-1-hydroxyet hyl]-4-methyl-7-oxo-1-azabicyclo[3.2.0]hept-2-ene-2-carboxy late (3)

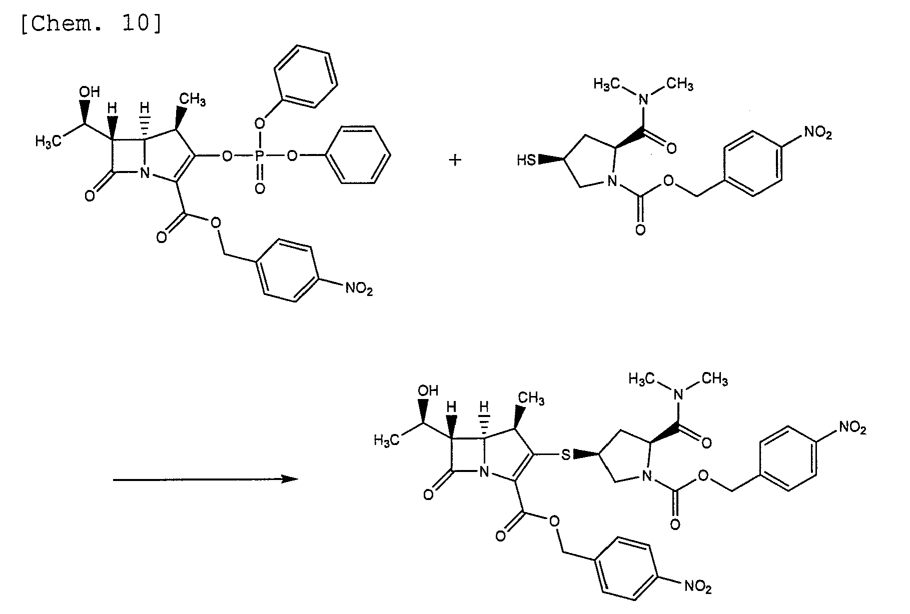

To 120 ml of acetonitrile were added 40.0 g of p-nitrobenzyl (4R,5R,6S)-3-diphenyloxyphosphoryloxy-6-[(1R)-1-hydroxyethy 1]-4-methyl-7-oxo-1-azabicyclo[3.2.0]hept-2-ene-2-carboxyla te and 25.0 g of (2S,4S)-2-dimethylaminocarbonyl-4-mercapto-1-(p-nitrobenzyl oxycarbonyl)pyrrolidine, and the mixture was cooled to -10°C with stirring. N,N-Diisopropylethylamine (10.5 g) was added thereto over 10 minutes, and the resulting mixture was stirred at the same temperature for 3 hours. To the reaction mixture were added, at 0 to 7°C, 240 ml of ethyl acetate and 200 ml of water, and the mixture was stirred for 10 minutes. The aqueous layer was removed, and the organic layer obtained was washed, at 0 to 10°C, with two 200-ml portions of a 10% aqueous solution of sodium chloride and one 200-ml portion of water, whereby a water-saturated ethyl acetate solution containing the compound (3) was obtained. The concentration of the compound (3) was 17% by weight.

### (Example 2)

### Production of (4R,5S,6S)-3-[[(3S,5S)-5-(dimethylaminocarbonyl)-3-pyrrolid inyl]thio]-6-[(1R)-1-hydroxyethyl]-4-methyl-7-oxo-1-azabicy clo[3.2.0]hept-2-ene-2-carboxylic acid (4)

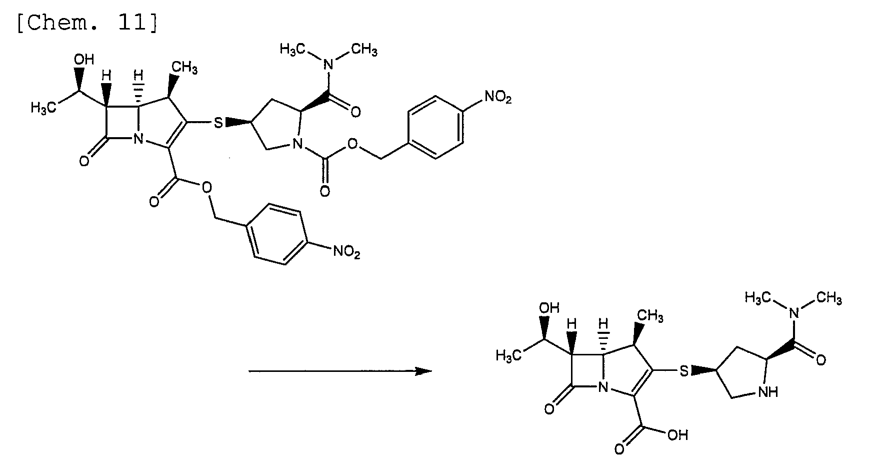

To 31.4 g of the water-saturated ethyl acetate solution containing the compound (3) as obtained in Example 1 were added 27.5 ml of n-butanol, 207 ml of water and 6.7 g of 10% Pd/C (50% hydrous), and the hydrogenation reaction was carried out at 33°C using hydrogen gas at atmospheric pressure. After 2.5 hours, the Pd/C was filtered off, the solution obtained was allowed to separate into two phases, and an aqueous solution containing 2.3 g of the compound (4) was obtained.

### (Example 3)

### Production of (4R,5S,6S)-3-[[(3S,5S)-5-(dimethylaminocarbonyl)-3-pyrrolid inyl]thio]-6-[(1R)-1-hydroxyethyl]-4-methyl-7-oxo-1-azabicy clo[3.2.0]hept-2-ene-2-carboxylic acid (4)

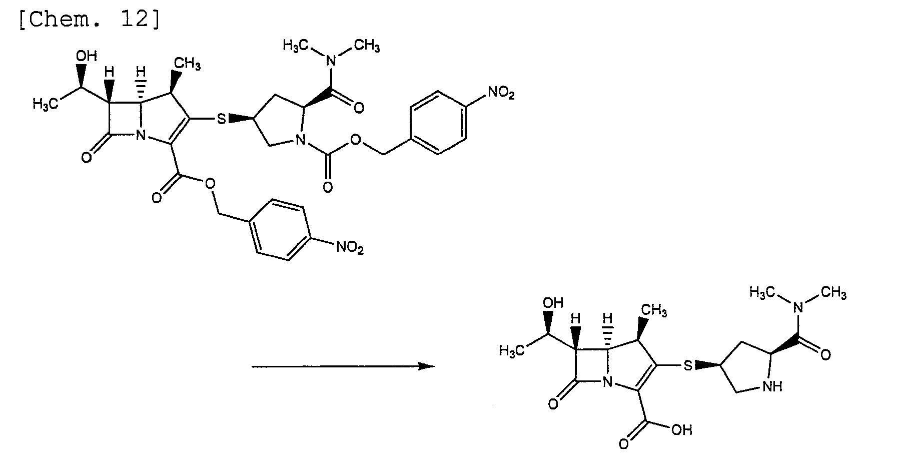

To 31.4 g of the water-saturated ethyl acetate solution containing the compound (3) as obtained in Example 1 were added 27.5 ml of ethyl acetate, 207 ml of water and 6.7 g of 10% Pd/C (50% hydrous), and the hydrogenation reaction was carried out at 33°C using hydrogen gas at atmospheric pressure. After 2.5 hours, the Pd/C was filtered off, 55 ml of n-butanol was added to the solution obtained, and the resulting mixture was allowed to separate into two phases, whereby an aqueous solution containing 2.1 g of the compound (4) was obtained.

### (Example 4)

### Production of trihydrate crystals (meropenem) of (4R,5S,6S)-3-[[(3S,5S)-5-(dimethylaminocarbonyl)-3-pyrrolid inyl]thio]-6-[(1R)-1-hydroxyethyl]-4-methyl-7-oxo-1-azabicy clo[3.2.0]hept-2-ene-2-carboxylic acid (4)

The aqueous solution containing the compound (4) as obtained in Example 2 was adjusted to pH 5.5 with a 10% aqueous solution of phosphoric acid with ice cooling and then concentrated to 75 g under reduced pressure. Active carbon (0.5 g) was added and, after 30 minutes of stirring with ice cooling, the mixture was filtered. The aqueous solution obtained was ice-cooled, seed crystals were added with stirring and, then, 317 ml of acetone was added over 2.5 hours. After further stirring for 1 hour, the precipitate crystals were collected by filtration and washed with 55 ml of acetone. The crystals obtained were dried at room temperature under reduced pressure to give 2.4 g of the trihydrate crystals of the compound (4).

¹H-NMR (D₂O): δ 1.22 (d, 3H, J = 7.1 Hz), 1.30 (d, 3H, J = 6.3 Hz), 1.92-1.99 (m, 1H), 3.03-3.11 (m, 1H), 3.00 (s, 3H), 3.07 (s, 3H) , 3. 34-3. 48 (m, 3H), 3.80 (dd, 1H, J = 6.3 Hz, 12.2 Hz) , 4.01-4.08 (m, 1H), 4.23-4.28 (m, 2H)
Water content (KF (Karl-Fischer) method): 12.3%
HPLC area percentage: 99.2%

### (Example 5)

### Production of p-nitrobenzyl (4R,5S,6S)-3-[[(3S,5S)-1-(p-nitrobenzyloxycarbonyl)-5-(dime thylaminocarbonyl)-3-pyrrolidinyl]thio]-6-[(1R)-1-hydroxyet hyl]-4-methyl-7-oxo-1-azabicyclo[3.2.0]hept-2-ene-2-carboxy late (3)

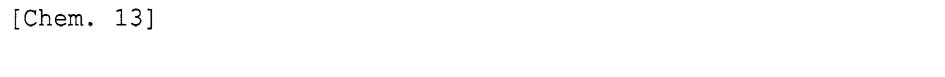

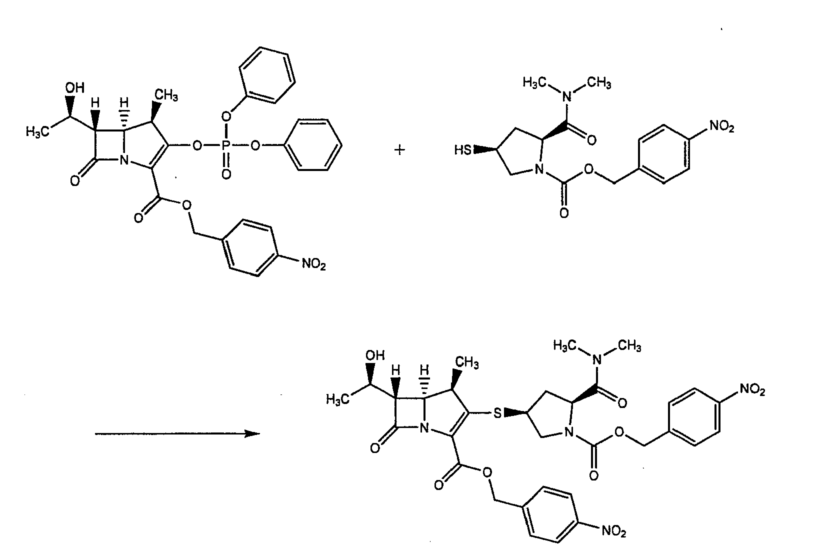

To 40 ml of dimethylacetamide were added 10.0 g of p-nitrobenzyl (4R,5R,6S)-3-diphenyloxyphosphoryloxy-6-[(1R)-1-hydroxyethy 1]-4-methyl-7-oxo-1-azabicyclo[3.2.0]hept-2-ene-2-carboxyla te and 6.2 g of (2S,4S)-2-dimethylaminocarbonyl-4-mercapto-1-(p-nitrobenzyl oxycarbonyl)pyrrolidine, and the mixture was cooled to -10°C with stirring. N,N-Diisopropylethylamine (6.9 g) was added thereto over 15 minutes, and the resulting mixture was stirred at the same temperature for 1 hour. To the reaction mixture were added, at -10 to -5°C, 120 ml of ethyl acetate and 85 ml of water, and the mixture was stirred for 10 minutes. The aqueous layer was removed, and the organic layer obtained was washed, at 0 to 10°C, twice with a mixture of 36 ml of a 5% aqueous solution of sodium chloride and 9 ml of 2 N hydrochloric acid and once with 45 ml of water, whereby a water-saturated ethyl acetate solution containing the compound (3) was obtained. The concentration of the compound (3) was 12% by weight.

### (Example 6)

### Production of p-nitrobenzyl (4R,5S,6S)-3-[[(3S,5S)-1-(p-nitrobenzyloxycarbonyl)-5-(dime thylaminocarbonyl)-3-pyrrolidinyl]thio]-6-[(1R)-1-hydroxyet hyl]-4-methyl-7-oxo-1-azabicyclo[3.2.0]hept-2-ene-2-carboxy late (3)

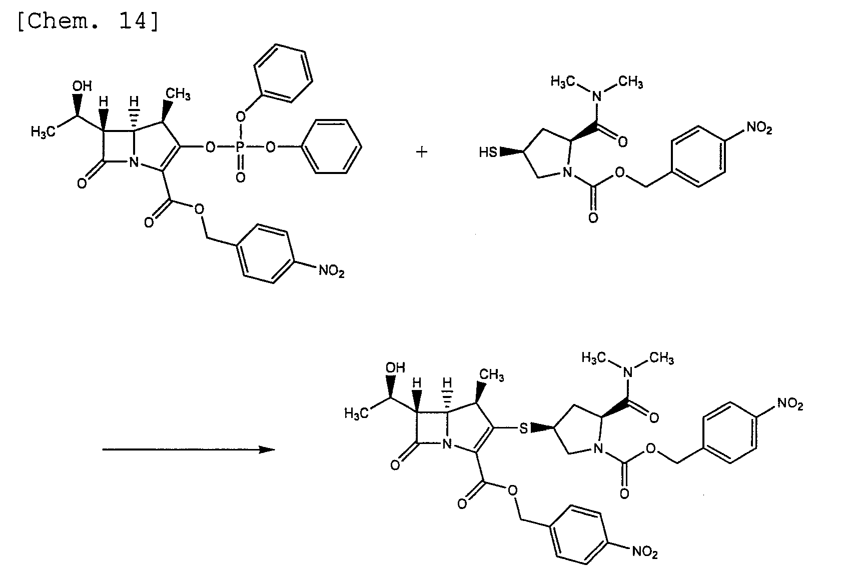

To 75 ml of dimethylformamide were added 24.8 g of p-nitrobenzyl (4R,5R,6S)-3-diphenyloxyphosphoryloxy-6-[(1R)-1-hydroxyethy 1]-4-methyl-7-oxo-1-azabicyclo[3.2.0]hept-2-ene-2-carboxyla te and 15.5 g of (2S,4S)-2-dimethylaminocarbonyl-4-mercapto-1-(p-nitrobenzyl oxycarbonyl)pyrrolidine, and the mixture was cooled to -10°C with stirring. N,N-Diisopropylethylamine (6.5 g) was added thereto over 10 minutes, and the resulting mixture was stirred at the same temperature for 3 hours. To the reaction mixture were added, at -10 to -5°C, 175 ml of ethyl acetate and 125 ml of water, and the mixture was stirred for 10 minutes. The aqueous layer was removed, and the organic layer obtained was washed, at 0 to 10°C, with two 125-ml portions of a 5% aqueous solution of sodium chloride and one 125-ml portion of water, whereby a water-saturated ethyl acetate solution containing the compound (3) was obtained. The concentration of the compound (3) was 18% by weight.

### (Example 7)

### Production of p-nitrobenzyl (4R,5S,6S)-3-[[(3S,5S)-1-(p-nitrobenzyloxycarbonyl)-5-(dime thylaminocarbonyl)-3-pyrrolidinyl]thio]-6-[(1R)-1-hydroxyet hyl]-4-methyl-7-oxo-1-azabicyclo[3.2.0]hept-2-ene-2-carboxy late (3)

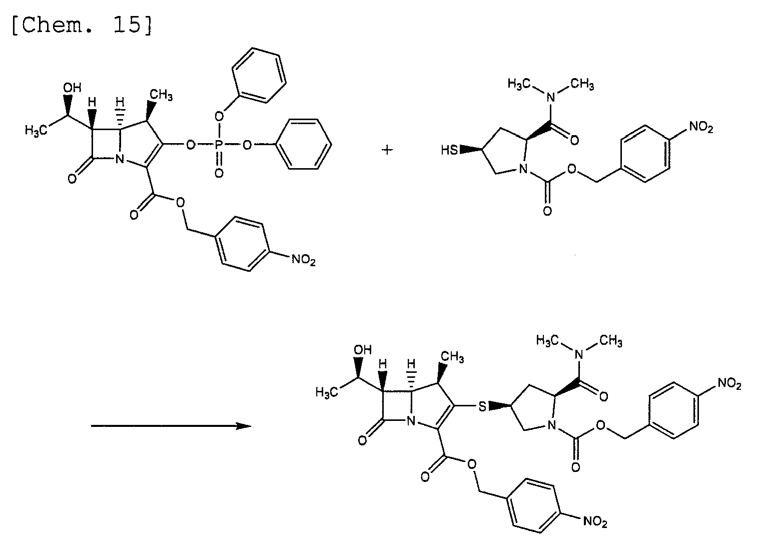

To a mixed solvent containing 20 ml of acetonitrile and 10 ml of ethyl acetate were added 10.0 g of p-nitrobenzyl (4R,5R,6S)-3-diphenyloxyphosphoryloxy-6-[(1R)-1-hydroxyethy 1]-4-methyl-7-oxo-1-azabicyclo[3.2.0]hept-2-ene-2-carboxyla te and 6.2 g of (2S,4S)-2-dimethylaminocarbonyl-4-mercapto-1-(p-nitrobenzyl oxycarbonyl)-pyrrolidine, and the mixture was cooled to -10°C with stirring. N,N-Diisopropylethylamine (2.6 g) was added thereto over 10 minutes, and the resulting mixture was stirred at the same temperature for 5 hours. To the reaction mixture were added, at 0 to 7°C, 50 ml of ethyl acetate and 50 ml of water, and the mixture was stirred for 10 minutes. The aqueous layer was removed, and the organic layer obtained was washed, at 0 to 10°C, with two 50-ml portions of a 5% aqueous solution of sodium chloride and one 50-ml portion of water, whereby a water-saturated ethyl acetate solution containing the compound (3) was obtained. The concentration of the compound (3) was 17% by weight.

### INDUSTRIAL APPLICABILITY

According to the process of the present invention, the compound (4) can be obtained without isolating/purifying the compound (3), which is the reaction intermediate. Therefore, the invention is especially advantageous in the mass production of the compound (4) on a commercial scale. The compound obtained is especially useful as an antimicrobial agent, and others.

## Claims

1. A process for producing a carbapenem compound represented by the general formula (4): 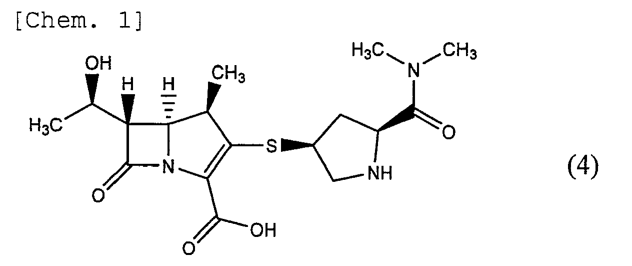 the process comprising
the step (A) of reacting a compound represented by the general formula (1): 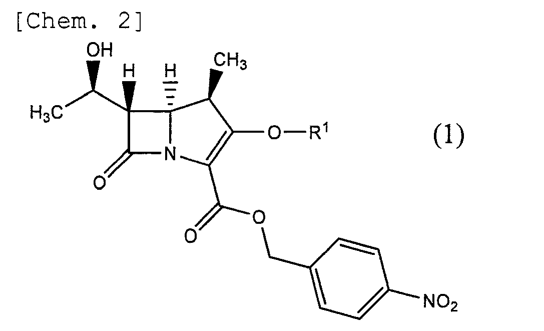 (wherein R¹ represents an acyl group) with a compound represented by the general formula (2): 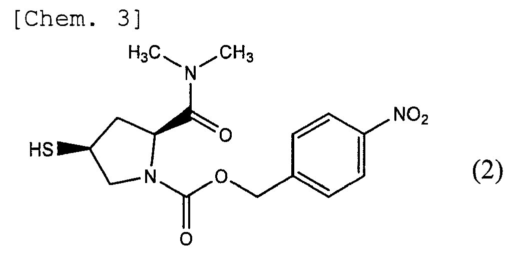 in an organic solvent in the presence of a base to obtain a compound represented by the general formula (3): 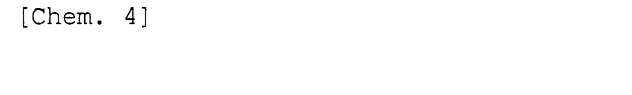 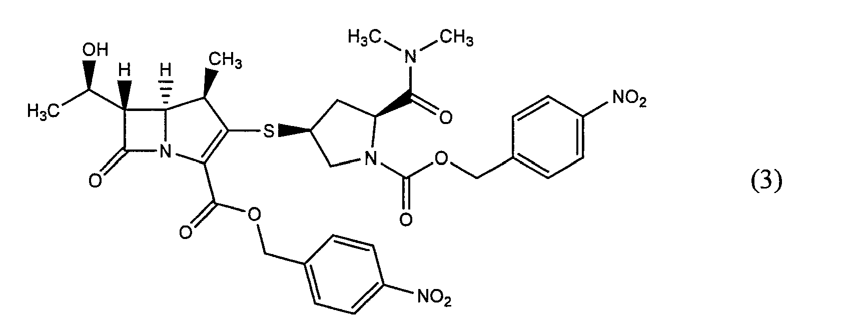 and
the step (B) of deprotecting the compound represented by the above formula (3) to obtain the compound represented by the above formula (4),
wherein the steps (A) and (B) are carried out without isolating the compound represented by the formula (3).

2. The process according to Claim 1,
wherein the organic solvent in the step (A) comprises at least one solvent selected from the group consisting of alcohols, ethers, esters, halogenated hydrocarbons, aromatic hydrocarbons, ketones, nitriles and amides.

3. The process according to Claim 1 or 2,
wherein the organic solvent in the step (A) comprises at least one solvent selected from the group consisting of acetonitrile, N,N-dimethylformamide, N,N-dimethylacetamide, N-methylpyrrolidone and N-ethylpyrrolidone.

4. The process according to any of Claims 1 to 3,
wherein the step (B) is carried out in a solvent comprising one or more organic solvents selected from the group consisting of alcohols, ethers, esters, halogenated hydrocarbons, aromatic hydrocarbons, ketones, nitriles and amides.

5. The process according to any of Claims 1 to 4,
wherein the deprotection reaction in the step (B) is hydrogenation in the presence of a palladium catalyst.

6. The process according to Claim 5,
wherein the deprotection reaction is carried out under the condition that the pH of an aqueous phase is 4 to 7.

7. A process for producing (4R,5S,6S)-3-[[(3S,5S)-5-(dimethylaminocarbonyl)-3-pyrrolid inyl]thio]-6-[(1R)-1-hydroxyethyl]-4-methyl-7-oxo-1-azabicy clo[3.2.0]hept-2-ene-2-carboxylic acid trihydrate represented by the general formula (5): 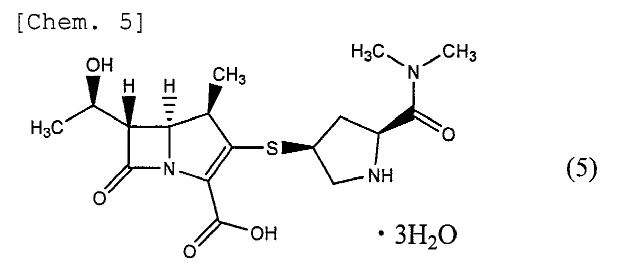 wherein an aqueous solution containing the compound represented by the formula (4) as obtained in any of Claims 1 to 6 is further subjected to the step of crystallization.

8. The process according to Claim 7,
wherein the crystallization is carried out by adding, to the aqueous solution, at least one organic solvent selected from the group consisting of ethanol, isopropyl alcohol, acetone, tetrahydrofuran, dioxane and acetonitrile.

9. The process according to Claim 8,
wherein the organic solvent is acetone.

10. The process according to any of Claims 1 to 9,
wherein R¹ is a diaryloxyphosphoryl group or a dialkoxyphosphoryl group.

11. The process according to Claim 10,
wherein R¹ is a diphenyloxyphosphoryl group.
